# EUROPÄISCHE PATENTSCHRIFT

(11) **EP 1 857 124 B1**
(45) Veröffentlichungstag und Bekanntmachung des Hinweises auf die Patenterteilung: **16.03.2011**
(21) Anmeldenummer: 06125777.0
(22) Anmeldetag: 11.12.2006
(51) Int. Cl.: A61L 2/16, A01N 37/02

(54) **Zusammensetzung zur Reinigung dentaler Instrumente und Verfahren**
Composition and method for cleaning dental instruments
Composition et méthode pour nettoyer les instruments dentaires

(30) Priorität: 15.05.2006 WO PCT/EP2006/062315
(43) Veröffentlichungstag der Anmeldung: 21.11.2007
(73) Patentinhaber: VOCO GmbH, 27472 Cuxhaven (DE)
(72) Erfinder: Barg, Andree, 21762, Otterndorf (DE); Plaumann, Manfred Thomas, 27476, Cuxhaven (DE)
(74) Vertreter: Eisenführ, Speiser & Partner

(56) Entgegenhaltungen:
- EP-A- 0 646 363
- WO-A-00/27438
- WO-A-96/20737
- DE-A1- 19 814 829
- US-A- 4 129 456
- US-A- 4 162 172
- US-A- 5 154 613
- ANONYMOUS: "Method for the removal of excess cement in the manufacture of dental restorations" RESEARCH DISCLOSURE, MASON PUBLICATIONS, HAMPSHIRE, GB, Bd. 461, Nr. 6, September 2002 (2002-09), XP007131120 ISSN: 0374-4353

## Beschreibung

Die Erfindung betrifft eine Zusammensetzung zur Reinigung dentaler Instrumente, insbesondere von Säure-Base-Zementresten, sowie ein entsprechendes Verfahren.

In der zahnärztlichen Praxis müssen Instrumente und Ausrüstung, wie Abdrucklöffel, Pinzetten, Zangen etc., die zur Untersuchung und Behandlung benötigt werden, von an der Oberfläche haftenden Verunreinigungen gereinigt werden. In der heutigen zahnmedizinischen Behandlung wird im Hinblick auf die prothetische Versorgung mit im Labor gefertigten Einlagearbeiten wie Kronen, Brücken, Inlays, Teilkronen, etc. ein Abdruck des Gebisses des Patienten genommen. Der Patient muss hierbei in einen sogenannten "Abdrucklöffel" (Abformlöffel) beißen, in den eine weiche Abdruckmasse gefüllt ist. Die Zähne des Gebisses verdrängen die Abdruckmasse, so dass sich eine negative Form ergibt, die aushärtet und vom Gebiss des Patienten abgezogen werden kann. Es können nun Füllmassen in die Negativform gegossen und ein positiver Abdruck hergestellt werden. Gefordert wird dabei eine exakte Wiedergabe der zu reproduzierenden Details.

Wird die Abdruckmasse aus dem Abdrucklöffel entfernt, so bleibt in der Regel ein Teil der ausgehärteten Abdruckmasse am Löffel haften. Die Reinigung des Abdrucklöffels ist ein schwieriger Arbeitsvorgang, weil einfaches manuelles Waschen unter Verwendung einer Bürste nicht ausreicht. Bedingt durch die geometrische Auslegung des Abdrucklöffels kann die Abdruckmasse in dessen Rillen oder Öffnungen haften, so dass ein Entfernen des Materials noch schwieriger zu bewerkstelligen ist.

Um der Schwierigkeit der Reinigung dentaler Abdrucklöffel aus dem Weg zu gehen, werden Einwegabdrucklöffel aus Kunststoff vermarktet. Nach dem Gebrauch wird der verunreinigte Löffel einfach weggeworfen. Die Verwendung von Einweg-Kunststoffabdrucklöffeln ist aber aus Kostengründen unwirtschaftlich und ökologisch nachteilig.

Gängigstes Material zur anatomischen Abformung von vollbezahnten, teilbezahnten und zahnlosen Kiefern sind Alginate, die wasserlöslichen Salze der wasserunlöslichen Alginsäure. Die Alginsäure ist eine Polyuronsäure und besteht aus 1,4-glykosidisch verknüpfter D-Mannuronsäure und L-Guluronsäure mit bis zu 750 Einheiten. Sie wird aus Rot- und Braunalgen gewonnen und in die entsprechenden Salze überführt. Das Prinzip der Alginatabformung besteht in der Überführung der wasserlöslichen Natrium-, Kalium- oder Ammoniumsalze in die wasserunlöslichen Calcium-, Blei- oder Bariumalginate durch Umsetzung der wasserlöslichen Salze mit Calciumsulfat oder geeigneten Blei- oder Bariumsalzen. Durch Auflösen eines Pulvers, das ein wasserlösliches Salz der Alginsäure und ein geeignetes weiteres Salz wie z. B. Calciumsulfat enthält, in Wasser wird der Abbindeprozess gestartet. Es bildet sich zunächst ein Sol, das schnell in den Gelzustand übergeht, da das wasserlösliche Salz der Alginsäure mit dem weiteren Salz reagiert und in irreversibler Weise als schwerlösliches Alginat, z. B. Calciumalginat, ausfällt. Chemisch betrachtet handelt es sich bei der Abbindereaktion somit um eine Ionenaustauschreaktion, bei der ein Platzwechsel der Metallionen an den Stellen stattfindet, an denen die Carboxylatgruppen des Alginats koordinativ abgesättigt werden.

Weitere wichtige Abdruckmaterialien sind Zinkoxid-Eugenol-Pasten. Diese Materialien sind Zweikomponentensysteme in Paste-Paste-Form, wobei die eine Komponente Zinkoxid und die andere Komponente Eugenol und Kolophonium (Abietinsäure) enthält. Die Abbindereaktion ist hier, ebenso wie im Fall der Alginate, eine Komplexierungsreaktion der Metallionen mit den Carboxylatgruppen. Zusätzlich komplexiert werden die Metallionen bei den Zinkoxid-Eugenol-Materialien durch die beiden Sauerstoffatome an den Positionen 1 und 2 des aromatischen Ringes von Eugenol.

Ähnliche Abbindemechanismen weisen Zinkphosphat- und Zinkpolycarboxylatzemente sowie Glasionomerzemente auf, die gängige Materialien im Dentalbereich sind.

Im Fall der Zinkphosphat- und Zinkpolycarboxylatzemente bilden ein basisches bis amphoteres Pulver und eine saure wässrige Lösung die Ausgangskomponenten. Bei den Zinkphosphatzementen wird Phosphorsäure mit Zinkoxid abgebunden. So führt die Reaktion zwischen einem Gewichtsteil Orthophosphorsäure und zwei Gewichtsteilen Zinkoxid zu dem kristallinen Reaktionsprodukt Hopeit, das über teilweise nebeneinander vorliegende Zwischenphasen von primären und sekundären Phosphaten erreicht wird. In der Praxis ist eine erfolgreiche Reinigung dentaler Instrumente von Verunreinigungen durch Zinkphosphatzemente besonders bedeutsam. Zum einen ist Zinkphosphatzement ein sehr häufig eingesetztes Material, zum anderen zeichnet er sich durch eine sehr hohe Haftfähigkeit aus, weshalb sich die Entfernung dieses Materials besonders aufwändig gestaltet. Im Fall der Zinkpolycarboxylatzemente wird Polyacrylsäure mit Zinkoxid abgebunden. Die gelösten Zinkionen werden durch die Polyacrylsäure komplexiert. Durch den Einbau der Zinkionen werden die Polymerketten elektrostatisch fixiert.

Eine ähnliche Reaktion findet auch bei den Glasionomerzementen statt. Hier wird Glaspulver mit einer wässrigen Lösung von Polyacrylsäure gemischt. Durch den Angriff der Säure freiwerdende Ionen des Glaspulvers reagieren mit der ungesättigten Polycarbonsäure. Die zur Aushärtung führende Abbindereaktion beruht auf dem Einbau von Metallionen des Glases in die Polycarbonsäureketten, die diese elektrostatisch fixieren (komplexieren). Infolge der "Säure-Base"-Reaktion bildet sich ein Hydrogelsalz als bindende Matrix. Unter dem Einfluss der Metallionen wird die Polycarbonsäure fest und die Abbindung wird eingeleitet. Durch die zunehmende Bindung der Metallionen an die Polycarbonsäureketten kommt es bei schwach exothermer Reaktion zur Aushärtung des Zementes.

In der Praxis werden die vorstehend genannten Materialien häufig unter den Begriff "Säure-Base-Zemente" zusammengefasst. Im zahnärztlichen Arbeitsbereich gibt es somit die als "Säure-Base-Zemente" bezeichneten Systeme Alginat, Zinkoxid-Eugenol, Zinkphosphat und Zinkpolycarboxylat sowie Glasionomer, die alle mit zahnärztlichen Instrumenten wie Abdrucklöffeln und Pinzetten verarbeitet werden.

Im Stand der Technik zur Reinigung dentaler Instrumente werden ausgehärtete, fest an der Oberfläche der Instrumente anhaftende Reste von "Säure-Base"-Zementen mit Hilfe konzentrierter Reinigungslösungen oder Reinigungspulver aufwändig gesäubert, sofern nicht Einweginstrumente benutzt werden.

Üblicherweise verwendete Reinigungsmittel enthalten in der Regel einen zur Komplexierung von Metallionen geeigneten Chelatbildner (Ionen komplexierende Verbindung, Metallchelator) wie etwa Nitrilotriacetat (NTA), Zitronensäure oder EDTA. Insbesondere Aminocarboxylate (z.B. EDTA, NTA) entfalten ihre komplexierende Wirkung am vollständigsten bei einem pH-Wert von ca. 11 oder darüber. Die Reinigungsmittel des Standes der Technik weisen daher in der Regel einen entsprechend hohen pH-Wert auf.

Vielfach bestehen Abdrucklöffel jedoch aus Aluminium, das bei solchen stark alkalischen pH-Werten korrodiert. Zur Materialschonung von aus Aluminium bestehenden Instrumenten sollte der pH-Wert daher, wie eigene Untersuchungen jetzt zeigten, nicht über 9,5, vorzugsweise bei 8,5, liegen. In diesem schwach alkalischen pH-Bereich ist die Reinigungswirkung der üblicherweise eingesetzten Reinigungsmittel jedoch gering. Bei der Einstellung des pH-Wertes eines Reinigungsmittels, das auch für Aluminium geeignet ist, ergibt sich somit ein Zielkonflikt.

Es wurde bereits mehrfach versucht, die Reinigung zahnärztlicher Instrumente, z.B. metallischer Abdrucklöffel, von "Säure-Base"-Zementen zu verbessern. Diese Vorschläge beruhen vielfach auf dem Versuch, die in den Abbindereaktionen gebildeten Verbindungen chemisch abzubauen:
Das Dokument JP 61078706 gibt an, durch eine wässrige Lösung bestimmter Konzentration von Natriumcarbonat und Wasserstoffperoxid an einem Abformlöffel anhaftendes Calciumalginat vollständig abzubauen. Wasserstoffperoxid ist ein starkes Oxidationsmittel, für dessen Anwendung aufgrund seiner ätzenden und brandfördernden Eigenschaften spezielle Sicherheitsvorkehrungen und Unterweisungen der handhabenden Personen erforderlich sind. Der Einsatz eines solchen starken Oxidationsmittels könnte auch ungünstig sein hinsichtlich einer möglichen Korrosion metallischer Instrumente.
Das Dokument JP 07265335 offenbart eine Dampfbehandlung eines Abdrucklöffels bei 100°C bis 130°C, um den Löffel so innerhalb kurzer Zeit von verunreinigendem Material zu reinigen. Nachteilig an diesem Verfahren ist jedoch offensichtlich der hohe apparative Aufwand.

Die Druckschrift JP 08003588 beschreibt ein Reinigungsmittel, das durch Mischen von einem metallchelatisierenden Mittel wie EDTA und einem Alkalimetallsalz eines Diaminoethylglycerinderivates erhalten wird und das Alginat auflöst, ohne den Löffel zu korrodieren.

JP 2003165997 beschreibt eine Zusammensetzung, die Peroxyhydrate wie Natriumperborat und/oder Wasserstoffperoxid und eine Azolverbindung wie Benzothiazol umfasst. Die Löffelreinigungswirkung wird verbessert, wenn wenigstens ein Alkalimetallsalz, ausgewählt aus der Gruppe der Carbonate, Hydrogencarbonate, Phosphate, Sulfate oder Hydrogensulfate der Zusammensetzung zugefügt wird, wie z. B. Natriumcarbonat, Natriumhydrogencarbonat, Natriumphosphat oder Natriumsulfat. Die mit dem Einsatz von Wasserstoffperoxid verbundenen Nachteile wurden bereits oben erwähnt. Natrumperborat zerfällt im Wasser in Wasserstoffperoxid und Natriumhydrogenborat. Letztere Verbindung passiert Kläranlagen nahezu unverändert und kann so in Gewässer gelangen.

Zudem sei hingewiesen auf die folgenden Dokumente:
- D1:: WO 96/20737 A (UNIVERSITE DE MONTREAL; PREVOST, ANDRE; BARBEAU, JEAN; COTE, LUDGER; C) 11. Juli 1996 (1996-07-11)
- D2:: WO 00/27438 A (UNIVERSITE DE MONTREAL; BARBEAU JEAN ; GRAVEL, DENIS; HABI, ABDELKRIM) 18. Mai 2000 (2000-05-18)
- D3:: DE 198 14 829 A1 (MERZ + CO. GMBH &; CO. KG) 7. Oktober 1999 (1999-10-07)
- D4:: US 4 129 456 A (LONGO ET AL) 12. Dezember 1978 (1978-12-12)
- D5:: EP 0 646 363 A (NIPPON SEIKI CO. LTD) 5. April 1995 (1995-04-05)
- D6:: ANONYMOUS: "Method for the removal of excess cement in the manufac- ture of dental restorations" RESEARCH DISCLOSURE, MASON PUBLICATIONS, HAMPSHIRE, GB, Bd. 461, Nr. 6, September 2002 (2002-09), ISSN: 0374-4353

Aufgabe der vorliegenden Erfindung ist es, den beschriebenen Nachteilen des Standes der Technik abzuhelfen und eine im Vergleich zum Stand der Technik, insbesondere bei neutralen bis schwach alkalischen pH-Werten von ca. 7,0 bis 9,5, wirksamere Reinigung dentaler Instrumente von Verunreinigungen zu ermöglichen, die insbesondere von Zinkphosphatzementen stammen. Die Reinigung soll im Vergleich zum Stand der Technik vorzugsweise einfacher und/oder schneller durchzuführen sein. Vorzugsweise sollte ein anzugebendes Reinigungsmittel breit einsetzbar sein, was zum einen bedeutet, dass vorzugsweise jede Art dentales Instrument, insbesondere eines aus Aluminium, mit diesem Mittel schadlos zu säubern sein sollte und zum anderen, dass vorzugsweise neben Zinkphosphatzementen auch andere "Säure-Base"-Zemente effektiv zu entfernen sein sollten.

Gemäß einem ersten Aspekt der vorliegenden Erfindung wird diese Aufgabe gelöst durch eine
Zusammensetzung zur Reinigung dentaler Instrumente von Säure-basen Zementen, umfassend oder (vorzugsweise) bestehend aus:
- (a): Nitrilotriessigsäure und/oder Nitrilotriacetat in einer Gesamtmenge von 20 bis 70 Gewichtsteilen, vorzugsweise 27,5 bis 70 Gewichtsteilen,
- (b): Zitronensäure und/oder deren Salzen in einer Gesamtmenge von 15 bis 45 Gewichtsteilen,
- (c): einem oder mehreren Alkalimetallcarbonaten und/oder -hydrogencarbonaten in einer Gesamtmenge von 0 bis 50 Gewichtsteilen, bei ausschließlichem Einsatz von Aminocarboxylaten vorzugsweise 0 bis 40 Gewichtsteilen, besonders vorzugsweise 0 bis 25 Gewichtsteilen, bei ausschließlichem Einsatz von Aminosäuren vorzugsweise 20 bis 50 Ge- wichtsteilen.
- (d): einem oder mehreren Tensiden in einer Gesamtmenge von 0 bis 5 Ge- wichtsteilen,
- (e): einem oder mehreren Tablettierungshilfsstoffen in einer Gesamtmenge von 0 bis 10 Gewichtsteilen,
- (f): einem oder mehreren Korrosionsinhibitoren in einer Gesamtmenge von 0 bis 5 Gewichtsteilen, und
- (g): einem oder mehreren sonstigen Additiven in einer Gesamtmenge von 0 bis 55 Gewichtsteilen
wobei die Komponenten (a), (b), (c), (d), (e), (f) und (g) insgesamt in einer Menge von 100 Gewichtsteilen vorliegen, und
wobei die Komponenten so ausgewählt sind, dass der pH einer Lösung hergestellt durch Vermischen der Zusammensetzung mit 4000 Gawichtsteilen Wasser im Bereich von 7,0 bis 9,5 liegt, bei ausschließlichem Einsatz von Aminocarboxylaten (also in Abwesenheit von Aminosäuren) vorzugsweise 7,5 bis 9,5.

Wasser zählt hierbei nicht zu den Additiven der Komponente (g) der Zusammensetzung.

Der Einsatz von Aminosäuren oder alpha-Hydroxysäuren bzw. deren Salzen als Chelatbildner ist zwar bereits aus dem Stand der Technik bekannt. Die Erfindung beruht aber nun auf der überraschenden Erkenntnis, dass bei der Kombination von bestimmten Aminosäuren (bzw. deren Salzen) und bestimmten alpha-Hydroxysäuren (bzw. deren Salzen) eine synergistische Erhöhung der Reinigungskraft erzielt werden kann, die Kombination dieser Bestandteile also eine Reinigungswirkung besitzen kann, die über die bloße Addition der Reinigungswirkungen der einzelnen Komponenten hinausgeht. Diese überraschend effektive Reinigungswirkung wird insbesondere bei im Vergleich zum Stand der Technik nur neutralen bzw. schwach basischen pH-Werten erreicht (pH 7,0 bis 9,5). In diesem pH-Bereich werden dentale Instrumente, insbesondere solche aus Aluminium, durch die Reinigungslösung nicht korrodiert.

Der der Erfindung zugrundeliegende Synergieeffekt ist aus dem Stand der Technik nicht bekannt. Die Kombination von Aminosäuren (bzw. deren Salzen) und alpha-Hydroxysäuren (bzw. deren Salzen), insbesondere von Nitrilotriacetat bzw. Nitrifatriessigsäure und Zitronensäure bzw. Trinatriumzitrat in einem Reinigungsmittel, mit dem insbesondere dentale Instrumente von "Säure-Base"-Zementresten befreit werden sollen, ist auch nicht bekannt.

Gerade die Verwendung von Aminosäuren, die zum Teil eine sehr schlechte Wasserlöslichkeit haben, ist im erfindungsgemäßen Zusammenhang nicht bekannt. Erstaunlicherweise liefern diese in Wasser schlecht löslichen Verbindungen sehr gute Resultate bezüglich ihrer Wasserlösungsgeschwindigkeit. Vermutlich erfolgt ihre rasche Auflösung in Wasser aufgrund einer Neutralisation der Säuren durch die als Komponente (c) in der Mischung vorliegenden Metallcarbonate und Metallhydrogencarbonate.

Bei Verwendung von (regelmäßig schwer löslichen) Aminosäuren ist die Langerstabilität einer entsprechenden Zusammensetzung vergleichsweise hoch, vermutlich auf Grund der geringeren Hygroskopie der Aminosäuren im Verhältnis zu den Salzen der Aminosäuren. In manchen bevorzugten Fällen umfasst Komponente (a) ausschließlich Aminosäuren, also keine Aminocarboxylate. In anderen Fällen umfasst Komponente (a) ausschließlich Aminocarboxylate.

WO 00/27438 A offenbart bestimmte Zusammensetzungen zur Entfernung von Biofilmen, jedoch keine Zusammensetzungen, die neben vergleichsweise großen Mengen an Aminosäure/-carboxylat und alpa-Hydroxysäure/-salz lediglich 0-5 Gewichtsprozent Tenside umfassen.

DE 196 03 977 lehrt den Gebrauch einer Desinfektionslösung und einer davon unabhängigen Reinigungslösung in einem Verfahren zur Reinigung und Desinfektion von empfindlichen medizinischen Geräten, insbesondere von Endoskopen. Da Endoskope in erster Linie mit Resten von menschlichem Gewebe und Blut verunreinigt sind, stellt sich dort ein ganz anderes Reinigungsproblem als bei der Entfernung von ,,Säure-Base"-Zementresten von dentalen Instrumenten. Gemäß einer Ausführungsform enthält die offenbarte Desinfektionslösung Nitrilotriacetat und Zitronensäure in gleichen Gewichtsanteilen. Ein Grund für den gleichzeitigen Einsatz beider Substanzen wird nicht angegeben. Insbesondere wird kein synergistisches Zusammenwirken beschrieben. DE 196 03 977 offenbart keine Zusammensetzung mit einem pH im Bereich von 7,0 bis 9,5. Die in dieser Entgegenhaltung angegebenen bevorzugten pH-Bereiche liegen unter 7, insbesondere zwischen 4,5 und 6,5. Da der pK-Wert der letzten Protonierungsstufe von Zitronensäure bei 6,4 liegt, liegt die Zitronensäure in einem derar tig niedrigen pH-Bereich einfach protoniert vor, weshalb zu erwarten ist, dass ihre chelatisierende Wirkung beeinträchtigt und ein synergistisches Zusammenwirken mit Nitrilotriacetat bei der Entfernung von Zementresten nicht mehr gegeben wäre. Die überraschend hohe Reinigungskraft einer Zusammensetzung gemäß der vorliegenden Erfindung erlaubt es, die dentalen Instrumente bei Raumtemperatur von "Säure-Base"-Zementresten zu reinigen, während DE 196 03 977 für andere Verunreinigungen einen Temperaturbereich von 55 bis 65 °C lehrt.

Die Patentschrift DE 198 14 829 offenbart ein Reinigungs- und Desinfektionsmittel für ärztliche Instrumente wie chirurgisches Instrumentarium und Anästhesiematerial, insbesondere für Endoskope. Gelehrt wird der Einsatz von Zitronensäure in etwa dreifachem Gewichtsüberschuss über Nitrilotriacetat. Hervorgehoben wird die Rolle der Zitronensäure bei der Einstellung des pH-Werts der Zusammensetzung, der zwischen 1 und 5 liegt. Eine Begründung für den gleichzeitigen Einsatz von Zitronensäure und Nitrilotriacetat wird nicht gegeben. Wie schon oben für die DE 196 03 977 diskutiert, ist bei dem in DE 198 14 829 gelehrten pH-Bereich und Gewichtsverhältnis von Zitronensäure von Nitrilotriacetat keine synergistische Verstärkung der Reinigungswirkung der beiden Substanzen zu erwarten, wenn sie auf das nicht-verwandte Reinigungsproblem der Entfernung von Zementresten von dentalen Instrumenten angewandt würden. In der DE 198 14 829 wird außerdem ein Arbeiten im Temperaturbereich von 50 bis 70 °C gelehrt.

Es sind Zusammensetzungen gemäß dem ersten Aspekt der vorliegenden Erfindung bevorzugt, bei denen das Gewichtsverhältnis der Gesamtmenge von Nitrilotriessigsäure und Nitrilotriacetat zu der Gesamtmenge an Zitronensäure und deren Salzen in der Zusammensetzung größer ist als 1:1, vorzugsweise größer als 1,5 : 1, und dabei vorzugsweise kleiner als 5:1, bevorzugt kleiner als 3:1. Bei diesen Gewichtsverhältnissen ist die synergistische Verstärkung des Reinigungseffektes überraschenderweise besonders hoch.

Wie bereits erwähnt, kann beim gemeinsamen Einsatz von Aminosäuren und/oder deren Salzen, bevorzugt Nitrilotriessigsäure und/oder Nitrilotriacetat, und alpha-Hydroxysäuren und/oder deren Salzen, bevorzugt Zitronensäure und/oder Citrat, eine überraschenderweise synergistisch erhöhte Effektivität bei der Reinigung dentaler Instrumente von ,,Säure-Base"-Zementresten erzielt werden.

Ein zweiter Aspekt der vorliegenden Erfindung betrifft daher eine Zusammensetzung umfassend oder (vorzugsweise) bestehend aus
- (a): Nitrilotriessigsäure und/oder Nitrilotriacetat
- (b): Zitronensäure und/oder deren Salze,
- (c): einem oder mehreren Alkalimetallcarbonaten und/oder -hydrogencarbonaten in einer Gesamtmenge von 0 bis 50 Gewichtsteilen, bei Abwesenheit von Nitrilotriessigsäure vorzugsweise 0 bis 40 Ge- wichtsteilen, besonders vorzugsweise 0 bis 25 Gewichtsteilen, bei Abwe- senheit von Nitrilotriacetat vorzugsweise 20 bis 50 Gewichtsteilen,
- (d): einem oder mehreren Tensiden in einer Gesamtmenge von 0 bis 5 Ge- wichtsteilen,
- (e): einem oder mehreren Tablettierungshilfsstoffen in einer Gesamtmenge von 0 bis 10 Gewichtsteilen,
- (f): einem oder mehreren Korrosionsinhibitoren in einer Gesamtmenge von 0 bis 5 Gewichtsteilen und
- (g): gegebenenfalls einem oder mehreren sonstigen Additiven,
wobei die Komponenten (a), (b), (c), (d), (e), (f) und (g) insgesamt in einer Menge von 100 Gewichtsteilen vorliegen, und
wobei das Gewichtsverhältnis der Gesamtmenge von Komponente (a) zu der Gesamtmenge an Komponente (b) in der Zusammensetzung größer ist als 1:1, vorzugsweise größer als 1,5 : 1, und dabei vorzugsweise kleiner als 5:1, bevorzugt kleiner als 3:1.

Wasser zählt hierbei nicht zu den Additiven der Komponente (g) der Zusammensetzung.

DE 198 14 829 A1 offenbart keine Zusammensetzung mit einem erfindungsgemäßen Komponentenverhältnis.

Es wurde bereits darauf hingewiesen, dass ein pH-Wert im neutralen bis schwach alkalischen Bereich von 7,0 bis 9,5 für eine Zusammensetzung bevorzugt ist, mit der insbesondere Aluminiuminstrumente ohne Korrosionsgefahr gereinigt werden können. Erfindungsgemäß liegt daher der pH-Wert einer Lösung hergestellt durch Vermischen der Zusammensetzung gemäß dem zweiten Aspekt der vorliegenden Erfindung mit 4000 Gewichtsteilen Wasser im Bereich von 7,0 bis 9,5, bei Abwesenheit von Aminosäuren vorzugsweise im Bereich von 7,5 bis 9,5.

Die Zusammensetzungen gemäß dem ersten und zweiten Aspekt der vorliegenden Erfindung, insbesondere in ihren oben oder im Folgenden als bevorzugt bezeichneten Ausgestaltungen, enthalten vorzugsweise Komponenten wie Tenside, Tablettierungshilfsstoffe, Korrosionsinhibitoren und/oder sonstige Additive. Beispiele für bevorzugte sonstige Additive sind (weitere) Puffersubstanzen zur Einstellung und Stabilisierung des pH-Werts sowie weitere Chelatbildner.

Eine Zusammensetzung gemäß dem ersten oder zweiten Aspekt der vorliegenden Erfindung enthält vorzugsweise als Komponente (f) eine wirksame Menge im Bereich von größer als 0 bis maximal 5 Gewichtsanteilen an einem oder mehreren Korrosionsinhibitoren. Diese sind insbesondere wünschenswert, wenn metallische Instrumente mit unterschiedlichem Redoxpotential gleichzeitig gereinigt werden sollen. Hierfür ist in einer erfindungsgemäßen Zusammensetzung ein Anteil von mindestens 0,1 Gewichtsteilen an einem oder mehreren Korrosionsinhibitoren bevorzugt. Oberhalb einer gewissen Menge kann andererseits keine weitere Steigerung der Effizienz mehr beobachtet werden. Aus Kostengründen ist daher ein maximaler Anteil von 4 Gewichtsteilen Korrosionsinhibitoren bevorzugt.

Diese und alle in der Beschreibung folgenden Angaben von Gewichtsteilen sind jeweils unter der Maßgabe zu verstehen, dass die Komponenten (a), (b), (c), (d), (e), (f) und (g) insgesamt in einer Menge von 100 Gewichtsteilen vorliegen.

Beispiele für besonders wirksam als Korrosionsinhibitoren einsetzbare Filmbildner sind Benzotriazol/Tolyltriazol und/oder Alkylaminotriazol sowie ihre Salze. Bevorzugt ist auch der Einsatz von Natrium- oder Kaliumphosphat, But-2-in-1,4-diol, Tetrapolyphosphorsäuremethylester, Aminotrimethylenphosphonsäure, Hydroxyethan-1,1-diphosphonsäure, 2-Phosphonobutan-1,2,4-tricarbonsäure und Alkalisilikaten.

Der Einstellung des pH-Wertes mit Hilfe von Puffersubstanzen in Form der oder zusätzlich zu den Komponenten (a) und (b), die selbst als Puffersubstanzen bezeichnet werden können, kommt besondere Bedeutung zu. Wie oben ausgeführt, ist einerseits bekannt, dass Ionen komplexierende Verbindungen wie Aminocarboxylate (z.B. EDTA, NTA) ihre komplexierende Wirkung am vollständigsten bei einem pH-Wert von ca. 11 oder darüber entfalten, da bei niedrigeren pH-Werten eine vollständige Deprotonierung nicht erreicht werden kann. Andererseits bestehen zahnärztliche Instrumente, insbesondere auch Abformlöffel, aus den verschiedenartigsten Metallen, wie Stahl, Aluminium, Kupfer, Nickel sowie den entsprechenden Legierungen. Es war daher äußerst überraschend festzustellen, dass die erfindungsgemäßen Zusammensetzungen bei pH-Werten von nicht höher als 9,5 bei der Entfernung von "Säure-Base"-Zementresten sehr viel höhere Reinigungswirkungen erzielten als übliche Reiniger für "Säure-Base"-Zemente aus dem Stand der Technik. Sie sind daher einsetzbar als universelle Reinigungsmittel, geeignet zur Säuberung jeder Art von metallischen dentalen Instrumenten. Es war überraschend festzustellen, dass bei pH-Werten kleiner oder gleich 9,5, bei denen NTA nicht vollständig deprotoniert vorliegt, eine gute Reinigung von "Säure-Base"-Zementresten erzielt werden kann. Gleichzeitig werden Aluminiuminstrumente vorteilhafterweise nicht angegriffen.

Prinzipiell sind alle zur Anwendung bei der Reinigung dentaler Instrumente geeigneten Puffersubstanzen in Zusammensetzungen gemäß dem ersten oder zweiten Aspekt der vorliegenden Erfindung einsetzbar. Besonders bevorzugt ist aber bei Abwesenheit von Aminosäuren die Anwesenheit von 10 bis 25 Gewichtsteilen an einem oder mehreren Alkalimetallcarbonaten und/oder - hydrogencarbonaten als Komponente (c) in den Zusammensetzungen gemäß dem ersten oder zweiten Aspekt der vorliegenden Erfindung.

Beim Lösen einer erfindungsgemäßen Zusammensetzung ergibt sich durch die Wechselwirkung aller enthaltenen Puffersubstanzen ein komplexes Puffersystem. Zum Beispiel: Durch die Reaktion eines oder mehrerer Alkalimetallcarbonate und/oder -hydrogencarbonate (z. B. Natriumcarbonat, Natriumhydrogencarbonat, Kaliumcarbonat, Kaliumhydrogencarbonat oder Gemische davon) mit einer alpha-Hydroxysäure entstehen Kohlendioxid und Alkalimetallcarboxylate. Vom stöchiometrischen Umsatz der Edukte hängt der resultierende pH-Wert ab. Das System Alkalimetallcarbonat und/oder -hydrogencarbonat und alpha-Hydroxysäure stellt ein Puffersystem dar, wobei das Verhältnis zwischen Carbonat (und/oder Hydrogencarbonat) und Säure den pH-Wert bestimmt. Für den Fall, dass die Zusammensetzung als Feststoff vorliegt (siehe dazu unten), bestimmt die Menge dieser Komponenten ihre Auflösungsgeschwindigkeit in einer wässrigen Lösung. Als Puffersubstanz wirkt ebenfalls das Aminocarboxylat der Komponente (a) (Nitrilotriacetat), dessen Alkalimetallsalze basisch reagieren. Weitere gegebenenfalls enthaltene Bestandteile einer erfindungsgemäßen Zusammensetzung, beispielsweise Fettalkoholethoxylate als Tenside und Alkalimetallsalze des Benzotriazols als Korrosionsinhibitoren (siehe oben), können ebenfalls als Puffersubstanzen wirken.

Die Zitronensäure bzw. deren Salze der Komponente (b) dienen in den Zusammensetzungen gemäß dem ersten und zweiten Aspekt der vorliegenden Erfindung mehreren primären Zwecken, nämlich sowohl (i) der Komplexierung von Metallionen und somit der Reinigung als auch (ii) der Einstellung des pH-Wertes sowie (iii) bei Anwesenheit von Alkalimetallcarbonaten und/oder - hydrogencarbonaten der Komponente (c) gegebenenfalls dem Auflösungsprozess der Zusammensetzung. Die Aminosäure (Nitrilotriessigsäure) bzw. das Aminocarboxylat (Nitrilotriacetat) der Komponente (a) dienen sowohl (i) der stabilen Komplexierung von Metallionen, die bei der Auflösung der "Säure-Base"-Zementreste frei werden, und somit der Reinigung, als auch (ii) der Einstellung des pH-Wertes sowie (iiii), bei Verwendung der Aminosäure, dem Auflösungsprozess der Zusammensetzung. Es versteht sich, dass diese Zwecke sowie, und das ist besonders wichtig, ein synergistisches Zusammenwirken der Komponenten (a) und (b) bei der Komposition einer erfindungsgemäßen Zusammensetzung zu berücksichtigen sind. Bevorzugt ist der Einsatz einer Menge an Alkalimetallcarbonaten und/oder -hydrogencarbonaten, die ausreicht, um eine nahezu vollständige Deprotonierung der alpha-Hydroxysäure(n) bzw. der Aminosäure(n) zu erzielen.

Als Lösungsmittel für eine erfindungsgemäße Zusammensetzung eignen sich neben Wasser auch wässrige Lösungen, insbesondere solche, die Alkohole wie etwa Methanol, Ethanol, iso-Propanol, n-Propanol iso-Butanol, n-Butanol sec.-Butanol und/oder tert.-Butanol enthalten.

Bevorzugt sind Zusammensetzungen gemäß dem ersten oder zweiten Aspekt der vorliegenden Erfindung, die vorliegen als Tablette, Pulver, Granulat oder wässrige Lösung. Besonders bevorzugt ist eine erfindungsgemäße Zusammensetzung, die als Tablette vorliegt. Vorteile einer Tablette liegen insbesondere in der vereinfachten Handhabung, da auf umständliche Arbeitsschritte wie etwa Abwiegen oder Abmessen von Pulvern oder konzentrierten Reinigungslösungen verzichtet werden kann. Dies ermöglicht ein schnelleres Arbeiten und minimiert das Risiko eines unerwünschten Kontaktes mit dem Reinigungsmittel, insbesondere eines Einatmens pulverförmiger Substanzen. Zudem wird die Herstellung von Reinigungslösungen gleichbleibender Zusammensetzung erleichtert.

Die Verwendung sprudelnder Brausetabletten zum selbsttätigen Reinigen von Zahnprothesen ist bekannt und beispielsweise in den Patentschriften DE 695 32 420, DE 37 17 920, DE 38 88 503, DE 38 12 693, DE 100 54 693, DE 39 31 129, DE 42 00 002 und DE 39 34 390 beschrieben. Die technischen Anforderungen, Verschmutzungen von Zahnprothesen auf Kunststoffbasis chemisch/physikalisch mit Hilfe einer Reinigungsbrausetablette bzw. eines Reinigungsbrausepulvers zu entfernen, sind sehr unterschiedlich zu denen bei der Ablösung abgebundener "Säure-Base" Zementreste von zahnärztlichen Instrumenten wie metallischen Abdrucklöffeln. Während die Zusammensetzungen zur Reinigung von Zahnprothesen vorrangig fettige Speisereste sowie eiweißhaltige und schwerlösliche mineralische Ablagerungen aus dem Speichel vom Prothesenkunststoffmaterial zu entfernen haben, müssen die aktiven Bestandteile eines Mittels zur Reinigung zahnärztlicher metallischer Instrumente von festen "Säure-Base"-Zementresten anderen Anforderungen genügen. Insbesondere müssen hierbei die Metallionen aus ihren Komplexen mit den Säuren gelöst werden, um dann den festen Verbund der Säuren abbauen zu können.

Das gleichzeitige Vorliegen eines oder mehrerer Alkalimetallcarbonate und/oder -hydrogencarbonate (als gasbildende Substanzen, beispielsweise Natriumcarbonat, Natriumhydrogencarbonat, Kaliumcarbonat und Kaliumhydrogencarbonat) und einer alpha-Hydroxysäure und/oder einer Aminosäure in einer erfindungsgemäßen Zusammensetzung, z.B. Tablette, ist bevorzugt und führt beim Kontakt mit Wasser zu einem heftigen Aufsprudeln der entstehenden Lösung und einem beschleunigten Auflösungsprozess der Zusammensetzung. Die in den erfindungsgemäßen Zusammensetzungen vorliegenden alpha-Hydroxysäuren und/oder Aminosäuren dienen im Fall einer als Tablette vorliegenden erfindungsgemäßen Zusammensetzung bei Anwesenheit eines oder mehrerer Alkalimetallcarbonate und/oder-hydrogencarbonate auch als Sprengmittel.

Einer erfindungsgemäße Zusammensetzung in Tablettenform kann abhängig von der Größe der Tablette einen oder mehrere Tablettierungshilfsstoffe enthalten. Diese dienen unter anderem als Bindemittel zum Verkleben der einzelnen Komponenten in Tablettenform. Bei kleinen Tabletten sind geringe Mengen oder gar keine Tablettierungshilfsstoffe erforderlich, bei größeren Tabletten überproportional mehr. Bevorzugt ist ein Anteil von 1 bis 10 Gewichtsteilen eines oder mehrerer Tablettierungshilfsstoffe als Komponente (e) einer erfindungsgemäßen Zusammensetzung in Tablettenform. Die folgenden Listen geben geeignete Tablettierungshilfsstoffe an, bei denen es sich um Füllstoffe, Trockenbindemittel, Bindemittel für die Granulierung, Gleitmittel und Schmiermittel handeln kann und von denen einzelne oder mehrere eingesetzt werden können. Als Füllstoffe und Trockenbindemittel können Lactose, Saccharose, Mannit, Glycin, Leucin, Sorbit, mikrokristalline Cellulose, Stärke, Dicalciumphosphat und Polyglykole Verwendung finden. Geeignete Bindemittel für die Granulierung sind Stärke, Alginate, Polyvinylpyrrolidon und besonders Carboxymethylcellulose. Als Gleit- und Schmiermittel sind z.B. Stärke, Talkum, Siliciumdioxid, Magnesiumstearat und ähnliche Metallseifen brauchbar Ganz besonders bevorzugt ist eine Zusammensetzung gemäß dem ersten oder zweiten Aspekt der vorliegenden Erfindung, die als Tablette vorliegt und umfasst oder vorzugsweise besteht aus:
- (a): Nitrilotriessigsäure, Nitrilotriacetat oder deren Mischung in einer Gesamt- menge von 20 bis 70 Gewichtsteilen, vorzugsweise 30 bis 70 Gewichtstei- len,
- (b): Zitronensäure, deren Salzen oder einer Mischung von Zitronensäure und deren Salzen in einer Gesamtmenge von 15 bis 45 Gewichtsteilen,
- (c): einem oder mehreren Alkalimetallcarbonaten und/oder -hydrogen- carbonaten in einer Gesamtmenge von 10 bis 50 Gewichtsteilen, vorzugs- weise bei Abwesenheit von Nitrilotriessigsäure 10 bis 25 Gewichtsteilen und bei Abwesenheit von Nitrilotriacetat 20 bis 50 Gewichtsteilen,
- (d): einem oder mehreren Tensiden in einer Gesamtmenge von 0 bis 5 Ge- wichtsteilen,
- (e): einem oder mehreren Tablettierungshilfsstoffen in einer Gesamtmenge von 1 bis 10 Gewichtsteilen,
- (f): einem oder mehreren Korrosionsinhibitoren in einer Gesamtmenge von 0,1 bis 4 Gewichtsteilen, und
- (g): gegebenenfalls einem oder mehreren sonstigen Additiven,
wobei die Komponenten (a), (b), (c), (d), (e), (f) und (g) insgesamt in einer Menge von 100 Gewichtsteilen vorliegen,
wobei das Gewichtsverhältnis der Gesamtmenge von Nitrilotriessigsäure und Nitrilotriacetat zu der Gesamtmenge an Zitronensäure und ihren Salzen in der Zusammensetzung größer ist als 1:1, vorzugsweise größer als 1,5 : 1 und
wobei die Komponenten so ausgewählt sind, dass der pH einer Lösung hergestellt durch Vermischen der Zusammensetzung mit 4000 Gewichtsteilen Wasser im Bereich von 7,0 bis 9,5 liegt, bei Abwesenheit von Nitrilotriessigsäure vorzugsweise im Bereich von 7,5 bis 9,5.

Vorzugsweise ist das Gewichtsverhältnis der Gesamtmenge von Nitrilotriessigsäure und Nitrilotriacetat zu der Gesamtmenge an Zitronensäure und ihren Salzen in der Zusammensetzung dabei kleiner als 5:1, bevorzugt kleiner als 3:1.

Es sei noch einmal ausdrücklich darauf hingewiesen, dass Wasser hierbei nicht zu den Additiven der Komponente (g) der Zusammensetzung zählt. Neben den Komponenten (a), (b), (c), (d), (e), (f) und (g) kann die Zusammensetzung jedoch weitere Komponenten enthalten, insbesondere Wasser. Auch in diesem Fall gilt die Maßgabe, dass der pH einer Lösung hergestellt durch Vermischen einer solchen Zusammensetzung mit 4000 Gewichtsteilen Wasser im Bereich von 7,0 bis 9,5 liegt.

Besonders bevorzugt sind die folgenden als Tabletten vorliegenden Zusammensetzungen (A) und (B), umfassend oder bestehend aus:
(A)
   (a) Nitrilotriacetat-Natrium in einer Gesamtmenge von 24 bis 54 Gewichtsteilen, vorzugsweise 44 bis 54 Gewichtsteilen
   (b) Zitronensäure, Salze der Zitronensäure oder einer Mischung von Zitronensäure und deren Salze in einer Gesamtmenge von 22,5 bis 26,5 Gewichtsteilen, vorzugsweise Zitronensäure alleine
   (c) Natriumcarbonat in einer Gesamtmenge von 18 bis 22 Gewichtsteilen,
   (d) C₁₆C₁₈-Fettalkoholethoxylate in einer Gesamtmenge von 0,45 bis 0,55 Gewichtsteilen,
   (e) Polyethylenglykol 6000 in einer Gesamtmenge von 4,5 bis 5,5 Gewichtsteilen,
   (f) 5-Methylbenzotriazol-Natrium in einer Gesamtmenge von 0,9 bis 1,1 Gewichtsteilen.
(B)
   (a) Nitrilotriessigsäure in einer Gesamtmenge von 24 bis 54 Gewichtsteilen, vorzugsweise 34 bis 44 Gewichtsteilen
   (b) Zitronensäure, Salze der Zitronensäure oder einer Mischung von Zitronensäure und deren Salze in einer Gesamtmenge von 19,2 bis 23,2 Gewichtsteilen, vorzugsweise Zitronensäure alleine
   (c) Natriumcarbonat in einer Gesamtmenge von 32,5 bis 36,5 Gewichtsteilen,
   (d) C₁₆C₁₈-Fettalkoholethoxylate in einer Gesamtmenge von 0,45 bis 0,55 Gewichtsteilen,
   (e) Polyethylenglykol 6000 in einer Gesamtmenge von 4,5 bis 5,5 Gewichtsteilen,
   (f) 5-Methylbenzotriazol-Natrium in einer Gesamtmenge von 0,9 bis 1,1 Gewichtsteilen.

Die Zusammensetzungen gemäß dem ersten oder zweiten Aspekt der vorliegenden Erfindung können ein oder mehrere Tenside enthalten. Die Tenside können sich zwischen den verschiedenen Phasen des zu reinigenden Systems einlagern und die Oberflächenspannung des Wassers an der Grenzfläche "zahnärztliches Instrument" / "Säure-Base"-Zementrest herabsetzen.

Nichtionische Tenside weisen in wässriger Lösung hohe Dipolmomente auf und sind stark hydratisiert. Beispiele für nichtionische Tenside sind Alkylenoxidaddukte, wie sie durch Anlagerung von Ethylenoxid und/oder Propylenoxid an Fettalkohole, Fettsäuren, Fettsäureglyceride, Phenole, Fettamine und Alkylphenole erhalten werden können, wobei die endständigen Hydroxylgruppen dieser Polyglykoletherderivate auch verethert, verestert oder acetalisiert sein können.

Geeignet sind insbesondere
- Anlagerungsprodukte von Ethylenoxid im molaren Verhältnis von 2- bis 50-fach und/oder von Propylenoxid im molaren Verhältnis von 1- bis 5-fach an lineare und verzweigte Fettalkohole mit 8 bis 30 C-Atomen, an Fettsäuren mit 8 bis 30 C-Atomen und an Alkylphenole mit 8 bis 15 C-Atomen in der Alkylkette,
- Anlagerungsprodukte von Ethylenoxid im molaren Verhältnis von 1- bis 30-fach an Glycerin,
- Anlagerungsprodukte von Ethylenoxid im molaren Verhältnis von 5- bis 60-fach an Rizinusöl oder gehärtetes Rizinusöl,
- Polyolfettsäureester,
- alkoxylierte Triglyceride,
- Aminoxide, wie N-Kokosalkyl-N,N-dimethylaminoxid oder N-Talgalkyl-N,N-dihydroxyethylaminoxid,
- Ester aus Sorbitan und ein, zwei oder drei Fettsäuren mit 8 bis 22 C-Atomen und einem Ethoxylierungsgrad von 4 bis 20,
- Fettsäurezuckerester, insbesondere Ester aus Saccharose und ein oder zwei Fettsäuren mit 8 bis 22 C-Atomen, beispielsweise Sucrose Cocoate, Sucrose Dilaurate, Sucrose Distearate, Sucrose Laurate, Sucrose Myristate, Sucrose Oleate, Sucrose Palmitate, Sucrose Stearate, Sucrose Ricinoleate, etc.,
- Alkyl- und Alkenyloligoglykoside,
- Anlagerungsprodukte von Ethylenoxid an Fettsäurealkanolamide und Fettamine, und
- Anlagerungsprodukte von Ethylenoxid an Zuckerfettsäureester.

Anionische Tenside bestehen aus einem hydrophoben Rest und einer negativ geladenen hydrophilen Kopfgruppe, die das Tensid wasserlöslich macht. Geeignete Gruppen sind beispielsweise Carboxylat-, Sulfat-, Sulfonat- oder Phosphatgruppen, die dann an lipophilen Alkylgruppen mit etwa 8 bis 30 C-Atomen gebunden sind. Zusätzlich können in der Verbindung Glykol oder Polyglykolethergruppen, Ester-, Ether- und Amidgruppen sowie Hydroxylgruppen enthalten sein.

Beispiele für geeignete anionische Tenside sind Salze und Ester von Carbonsäuren, Alkylethersulfate, Alkylsulfate, Fettalkoholethersulfate, Sulfonsäuren und ihre Salze, Phosphorsäureester und ihre Salze und Acylaminosäuren und ihre Salze.

Ein in diesem Zusammenhang wichtiger Aspekt der in den erfindungsgemäßen Zusammensetzungen enthaltenen Metallionen bindenden Chelatbildner ist es, bei der Verwendung von hartem Wasser in die Lösung eingebrachte Metallionen, insbesondere Calciumionen zu binden. So kann die Bildung schwerlöslicher Salze mit den anionischen Tensiden verhindert und somit deren Reinigungswirkung erhalten werden. Neben der Bildung stabiler Komplexe mit den Metallionen aus den Produkten der Neutralisation der Zementreaktion ist es somit vorteilhaft, vorhandene Salze zu eliminieren, da salzhaltiges Wasser den Reinigungsvorgang erschwert und die Wirksamkeit der Tenside einschränkt.

Amphotere Tenside tragen sowohl eine negative als auch eine kompensierende positive Ladung.

Beispiele für amphotere Tenside sind die Derivate tertiärer aliphatischer Amine und quartärer aliphatischer Ammonium-Verbindungen, deren aliphatische Reste geradkettig oder verzweigt sein können und von denen einer eine Carboxy-, Sulfo-, Phosphono-, Sulfato-, oder Phosphatogruppe trägt, wie beispielsweise N,N-Dimethyl-N-tetradecylglycin, N,N-Dimethyl-N-hexadecylglycin, N,N-Dimethyl-N-octadecylglycin oder 3-(N,N-Dimethyl-N-dodecylammonium)-1-propansulfonat.

Weitere amphotere Tenside sind die Betaine. Beispiele hierzu umfassen die
- Alkylbetaine mit 8 bis 18 C-Atomen wie Cocodimethylcarboxymethylbetain,
- Lauryldimethylcarboxymethylbetain, Lauryldimethylcarboxyethylbetain, Cetyldimethylcarboxymethylbetain, Oleyldimethylgammacarboxypropylbetain und Laurylbis(2-hydroxypropyl)alphacarboxyethylbetain,
- Sulfobetaine mit 8 bis 18 C-Atomen wie Cocodimethylsulfopropylbetain, Stearyldimethylsulfopropylbetain, Lauryldimethylsulfoethylbetain, Laurylbis(2-hydroxyethyl)sulfopropylbetain,
- Carboxylderivate des Imidazols,
- Alkyldimethylammoniumacetate mit 8 bis 18 C-Atomen,
- Alkyldimethylcarbonylmethylammoniumsalze mit 8 bis 18 C-Atomen, und
- Fettsäurealkylamidobetaine mit 8 bis 18 C-Atomen.

Kationische Tenside bestehen aus einem hydrophoben Rest und einer positiv geladenen hydrophilen Kopfgruppe, die das Tensid wasserlöslich macht. Das positiv geladene Atom stellt in der Regel ein Stickstoffatom dar, an dem vier voneinander unabhängige Gruppen gebunden sind. Diese Gruppen können aliphatisch oder aromatisch sein, es können Alkoxygruppen, Polyoxyalkylengruppen, Alkylaminogruppen, Hydroxyalkylgruppen, Arylguppen oder Alkarylgruppen mit 1 bis 22 C Atomen sein, wobei mindestens ein Rest mindestens 6, besser noch 8 C Atome aufweist. Das positiv geladene Stickstoffatom muss durch ein Anion, beispielsweise ein Halogen, ein Acetat, ein Phosphat, ein Nitrat oder ein Alkylsulfat neutralisiert sein.

Beispiele für kationische Tenside sind quartäre Ammoniumverbindungen wie Ammoniumhalogenide, insbesondere Chloride und Bromide, also Alkyltrimethylammoniumchlorid, Dialkyldimethylammoniumchlorid, Trialkylmethylammoniumchlorid wie Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid sowie die entsprechenden Bromide.

Ferner geeignet sind Alkylpyridiniumsalze wie Lauryl- oder Cetylpyridiniumsalze oder Verbindungen, die sowohl mindestens eine Esterfunktion als auch mindestens eine quartäre Ammoniumgruppe als Strukturelement enthalten, beispielsweise quaternierte Estersalze von Fettsäuren mit Triethanolamin, quaternierte Estersalze von Fettsäuren mit Diethanolalkylaminen oder quaternierte Estersalze von Fettsäuren mit 1,2-Dihydroxypropyldialkylamin.

Ebenfalls geeignet sind Alkylamidoamine, die durch Amidierung von Fettsäuren mit Dialkylaminoaminen herstellbar sind.

Es können auch Gemische der oben genannten Tenside verwendet werden. Bevorzugt werden die nicht-ionischen Tenside aufgrund ihrer geringen Tendenz zum Schäumen eingesetzt, die die Handhabung erleichtert.

Wie ausgeführt, kann eine synergistische Verstärkung der Reinigungswirkung auf ,,Säure-Base"-Zementreste bei der Kombination von Chelatbildnem aus der Gruppe der Aminocarboxylate mit Chelatbildnem aus der Gruppe der alpha-Hydroxysäuren beobachtet werden. Beispiele für allgemein in Zusammensetzungen gemäß dem ersten oder zweiten Aspekt der vorliegenden Erfindung einsetzbare Chelatbildner sind zunächst die Aminocarboxylate (z.B. Nitrilotriacetat (NTAerfindungsgemäß), Ethylendiamintetraacetat (EDTA), Diethytantriaminpentaacetat, Hydroxyethylethylendiamintriacetat und Methylglycindiacetat) und deren Säuren und die alpha-Hydroxysäuren (z.B. Glykolsäure, Milchsäure, Mandelsäure, Äpfelsäure, Weinsäure, Zitronensäure (erfindungsgemäß)) und deren Salze. Darüber hinaus können als (Bestandteile der) Komponente (g) weitere Chelatbildner eingesetzt werden. Hierzu zählen Tetrakis(-2-hydroxypropyl)ethylendiamin, 1-Hydroxyethan-1,1-diphosphonsäure, Aminotris(methylenphosphonsäure), Ethylendiamintetrakis(methylenphosphonsäure), Phosphonobutantricarbonsäure, Glukonsäure, Adipinsäure, Fumarsäure, Bernsteinsäure und deren Salze sowie Gemische der Säuren und deren Salze. Beispiele weiterer geeigneter Chelatbildner umfassen Natriumtripolyphosphat, saures Natriumpyrophosphat und Tetranatriumpyrophosphat. Ebenfalls geeignet sind Polycarbonsäuren sowie polymere Polycarboxylate wie beispielsweise die Metallsalze der Polyacrylsäure oder der Polymethacrylsäure. Hierzu zählen auch copolymere Polycarbonsäuren und copolymere Polycarboxylate wie Copolymere der Acrylsäure mit Maleinsäure oder Copolymere der Acrylsäure mit Methacrylsäure. Diese Verbindungen können zusätzlich noch mit Allylsulfonsäuren wie beispielsweise mit Allyloxybenzolsulfonsäure oder Methallylsulfonsäure modifiziert werden. Andere Copolymere weisen Acrolein und Acrylsäure oder Acrolein und Vinylacetat als Strukturelemente auf.

Zusammensetzungen gemäß dem ersten oder zweiten Aspekt der vorliegenden Erfindung sind außer zur Entfernung von "Säure-Base"-Zementen auch zur Entfernung von Haftlack von zahnärztlichen Instrumenten geeignet. Besonders gut eignet sich eine erfindungsgemäße Zusammensetzung auch zur Reinigung sogenannter Schreinemakers-Löffel. Diese Löffel bestehen aus verchromten Zink, das durch ein Druckgussverfahren in seine Form gebracht wird. Solche Materialien werden oftmals leicht korrodiert, da es in der aufgetragenen Chromschicht leicht zu Mikrorissen kommen kann. Dies wird durch den pH-Bereich einer Reinigungsflüssigkeit enthaltend eine erfindungsgemäße Zusammensetzung und die vorzugsweise verwendeten Korrosionsinhibitoren weitgehend unterbunden. Außer zur Reinigung metallischer dentaler Instrumente sind die erfindungsgemäßen Zusammensetzungen selbstverständlich auch zur Reinigung von Kunststoffinstrumenten geeignet.

Aufgrund der hohen Reinigungswirkung der erfindungsgemäßen Zusammensetzungen ist eine Ultraschallbehandlung der zu reinigenden Instrumente nicht erforderlich. Ein Einsatz der Reinigungslösungen in einem Ultraschallbad ist jedoch problemlos möglich.

Vorteilhaft an den erfindungsgemäßen Zusammensetzungen ist überdies, dass sie biologisch abbaubar und nach OECD materialschonend sind.

Gemäß einem dritten Aspekt betrifft die vorliegende Erfindung auch ein Verfahren zur Herstellung einer wässrigen Reinigungsflüssigkeit für dentale Instrumente, umfassend den folgenden Schritt:
- Herstellen einer Mischung umfassend eine vorzugsweise gelöste erfindungsgemäße Zusammensetzung, wobei die Komponenten (a), (b), (c), (d), (e), (f) und (g) in einer Menge von insgesamt 100 Gewichtsteilen vorliegen und Wasser in einer Menge von maximal 10000, bevorzugt 2000 bis 6000 Gewichtsteilen vorliegt.

Ein Beispiel hierfür ist das Auflösen einer als Tablette vorliegenden erfindungsgemäßen Zusammensetzung in Wasser oder einer wässrigen Lösung.

Weiterhin betrifft die vorliegende Erfindung gemäß einem vierten Aspekt eine wässrige Reinigungsflüssigkeit für dentale Instrumente, umfassend
- eine gelöste oder dispergierte erfindungsgemäße Zusammensetzung, wobei die Komponenten (a), (b), (c), (d), (e), (f) und (g) in einer Menge von insgesamt 100 Gewichtsteilen vorliegen, und
- Wasser in einer Menge von maximal 10000, bevorzugt 2000 bis 6000 Gewichtsteilen.

Ein weiterer Aspekt der vorliegenden Erfindung betrifft die Verwendung von Zitronensäure und/oder deren Salzen zur synergistischen Verstärkung der "Säure-Base"-Zementreste entfernenden Wirkung von Nitrilotriacetat, Nitrilotriessigsäure oder deren Mischung. Ein eng verwandter Aspekt betrifft auch die Verwendung von Nitrilotriacetat, Nitrilotriessigsäure oder deren Mischung zur synergistischen Verstärkung der "Säure-Base"-Zementreste entfernenden Wirkung von Zitronensäure und/oder deren Salzen.

Weitere Aspekte der Erfindung ergeben sich aus den beigefügten Patentansprüchen und den nachfolgenden Beispielen.

Die Beispiele sollen die vorliegende Erfindung verdeutlichen, ohne diese einzuschränken.

### Beispiele 1 bis 16: Zusammensetzungen zur Reinigung dentaler Instrumente

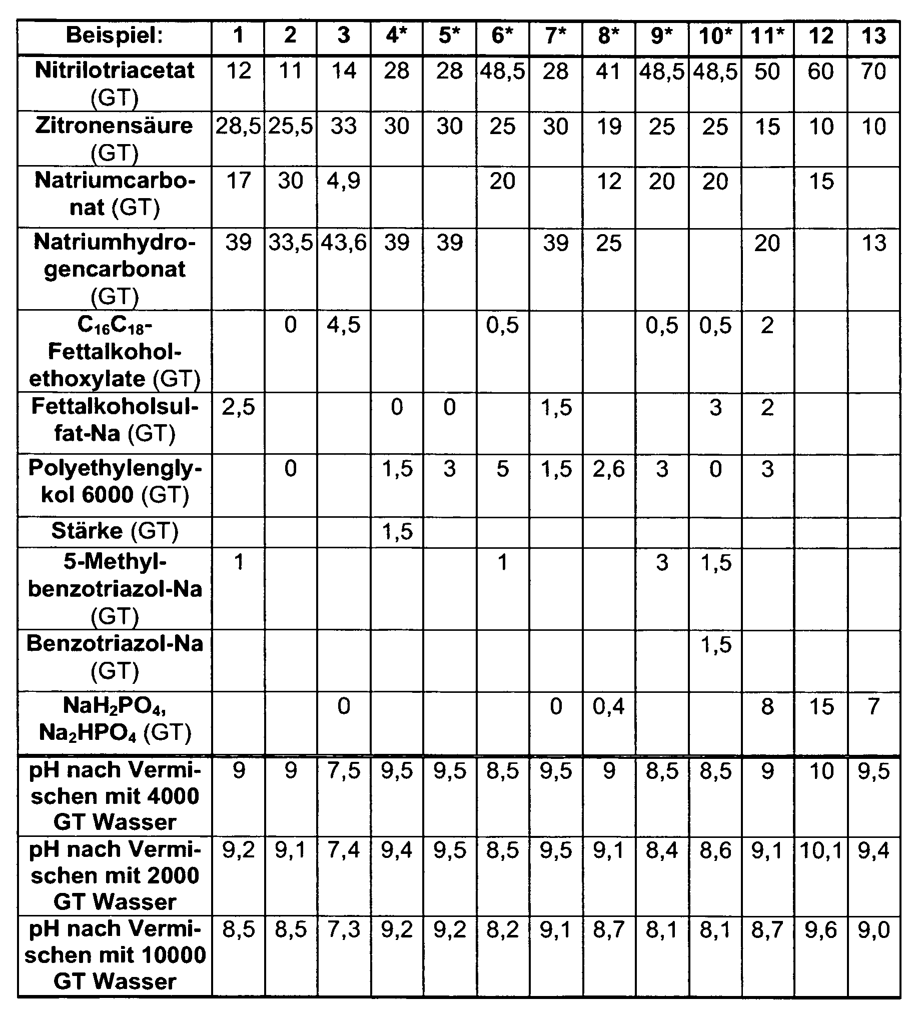

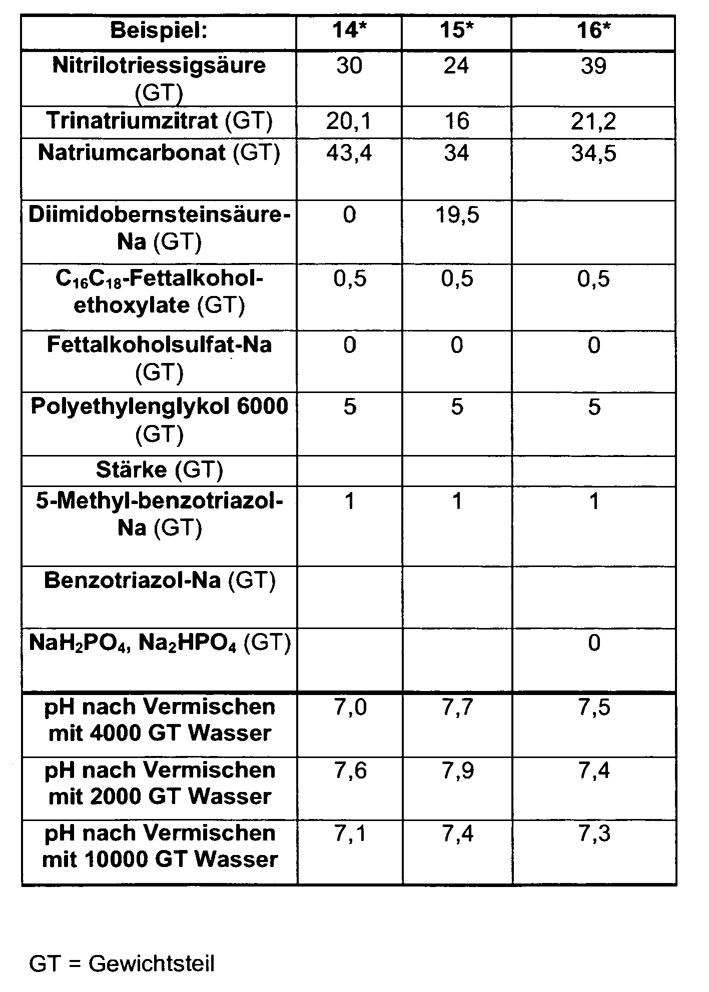

Bei den mit einem Stern * gekennzeichneten Beispielen 4, 5, 6, 7, 8, 9, 10, 11, 14, 15 und 16 handelt es sich um erfindungsgemäße Beispiele, bei den Beispielen 1, 2, 3, 12 und 13 um Vergleichsbeispiele.

In jedem Beispiel 1 bis 16 beträgt die Summe der Gewichtsteile (GT) 100.

Die Zusammensetzungen lagen jeweils als Feststoff (Pulver) vor.

Die Lösungen hergestellt durch Vermischen der Zusammensetzungen mit der genannten Zahl an Gewichtsteilen Wasser hatten die angegebenen pH-Werte.

### Beispiel 17: Tablette

Eine Zusammensetzung nach Beispiel 6 wurde als Tablette hergestellt. Dazu wurden die gut vermischten Bestandteile in einen Stahlzylinder (Durchmesser 40 mm, Höhe 60 mm) gefüllt und mittels eines Stempels verpresst. Durch eine Hydraulik wirkte eine Gewichtskraft von 3 t auf den Stempel.

Analog wurde eine Zusammensetzung nach Beispiel 16 als Tablette hergestellt.

### Beispiel 18: Methode zur quantitativen Bestimmung einer Reinigungswirkung (vgl. auch die Beispiele 19, 20 und 21)

Es wurden für jede zu untersuchende Reinigungslösung identische scheibenförmige Prüfkörper (Durchmesser 2 cm +/- 0,2 mm, Stärke 2 mm +/- 0,1 mm) eines bestimmten Zements (siehe unten sowie Beispiele 19, 20 und 21) hergestellt und gewogen. Wässrige Reinigungslösungen (siehe Beispiele 19, 20 und 21) wurden durch Auflösen von jeweils 100 Gewichtsteilen bestimmter Zusammensetzungen in 4000 Gewichtsteilen Wasser hergestellt. Die identischen Prüfkörper wurden jeweils mittels eines Netzes (Maschenweite 500 µm) in 250 ml der jeweiligen wässrigen Reinigungslösung (20°C) gehängt.

Nachdem die Prüfkörper 3 h (z.B. Alginat, Zinkpolycarboxylat) bzw. 8 h (z.B. Zinkphosphat) in die jeweilige Lösung getaucht worden waren, wurde die Lösung durch eine tarierte Glasfritte abgesaugt, der Rückstand dreimal mit je 20 ml Wasser gewaschen, bei 150 °C bis zur Massenkonstanz getrocknet und gewogen. Die Masse des jeweiligen Rückstandes geteilt durch die eingesetzten, jeweils identischen Prüfkörpermasse stellt ein Maß für die Reinigungswirkung dar. Man beachte, dass in diesen Versuchen lediglich die von den Füllstoffen resultierende abgesetzte und wasserunlösliche Masse am Boden der Lösung zur Ermittlung der Reinigungseffizienz berücksichtigt wird.

### Beispiel 19: Wässrige Reinigungsflüssigkeiten

Wässrige Reinigungsflüssigkeiten für dentale Instrumente wurden durch Vermischen von Zusammensetzungen gemäß Beispielen 1 bis 16 sowie Beispiel 17 (Tablette) mit 4000 GT Wasser hergestellt.

19.1: Die Reinigungswirkungen von Reinigungsflüssigkeiten auf Basis sämtlicher Zusammensetzungen gemäß Beispiel 1 bis 16 wurden gegenüber jeweils identischen Prüfkörpern aus Zinkphosphat-Zement quantifiziert. Hierzu wurde die Methode aus Beispiel 18 angewendet. Es zeigte sich, dass den erfindungsgemäßen Beispielen 4 bis 11 und 14 bis 16 entsprechende Reinigungsflüssigkeiten den Vergleichsbeispielen 1 bis 3 sowie 12 bis 13 entsprechenden Reinigungsflüssigkeiten überlegen waren. Weiterhin zeigte sich, dass die An- oder Abwesenheit von Carbonaten bzw. Hydrogencarbonaten keinen signifikanten Unterschied in der Reinigungswirkung zur Folge hatte; entscheidend waren die Mengen an Nitrilotriacetat und Zitronensäure.

19.2: Die Reinigungswirkungen von Reinigungsflüssigkeiten auf Basis der erfindungsgemäßen Beispiele 4 bis 11 und 14 bis 16 wurde gegenüber Alginat (demedis), Zinkphosphatzement (Poscal, VOCO) und Zinkpolycarboxylatzementen (Carboco, VOCO) getestet. Die Zemente wurden jeweils nach Herstellerangaben zubereitet. Aluminium-Abdrucklöffel wurden mit je 10 g Alginat-Abformmasse gleichmäßig bestrichen. Dentalinstrumente wurden mit je 1 g Zinkphosphatzement bzw. je 1 g Zinkpolycarboxylatzement gleichmäßig bestrichen. Nach vollständigem Ablauf der Abbindereaktion wurden die in der genannten Weise verschmutzten Abdrucklöffel bzw. Instrumente 6 h bei 20°C in den wässrigen Reinigungsflüssigkeiten inkubiert und danach unter fließendem kalten Wasser abgespült. In allen Fällen wurden die Verschmutzungen durch dieses Vorgehen vollständig entfernt. Bei mikroskopischer Untersuchung wurden keine Hinweise auf eine Korrosion der Löffel gefunden.

### Beispiel 20: Synergistischer Effekt

Es wurden die Zusammensetzungen A, B, C, D und E gemäß folgender Tabelle hergestellt und in jeweils 4000 Gewichtsteilen (GT) Wasser bei 20°C gelöst. Der pH-Wert wurde dann mit HCI bzw. NaOH auf jeweils 8,5 eingestellt.

| **Zusammensetzung:** | **A** | **B** | **C*** | **D*** | **E** |
|---|---|---|---|---|---|
| **Nitrilotriacetat** (GT) | 0 | 40 | 67 | 80 | 100 |
| **Zitronensäure (GT)** | 100 | 60 | 33 | 20 | 0 |
| **pH nach Vermischen mit 4000 GT Wasser** | 8,5^{#} | 8,5^{#} | 8,5^{#} | 8,5^{#} | 8,5^{#} |

| | | | | | |
|---|---|---|---|---|---|
| ^{#} pH mit HCl, bzw NaOH eingestellt GT = Gewichtsteil | | | | | |

Bei den mit einem Stern * gekennzeichneten Beispielen C und D handelt es sich um erfindungsgemäße Beispiele, bei den Beispielen A, B und E um Vergleichsbeispiele.

Die Reinigungswirkung der unterschiedlichen Lösungen wurde gegenüber jeweils identischen Prüfkörpern aus Zinkphosphat-Zement (Poscal, VOCO) gemäß Beispiel 18 bestimmt.

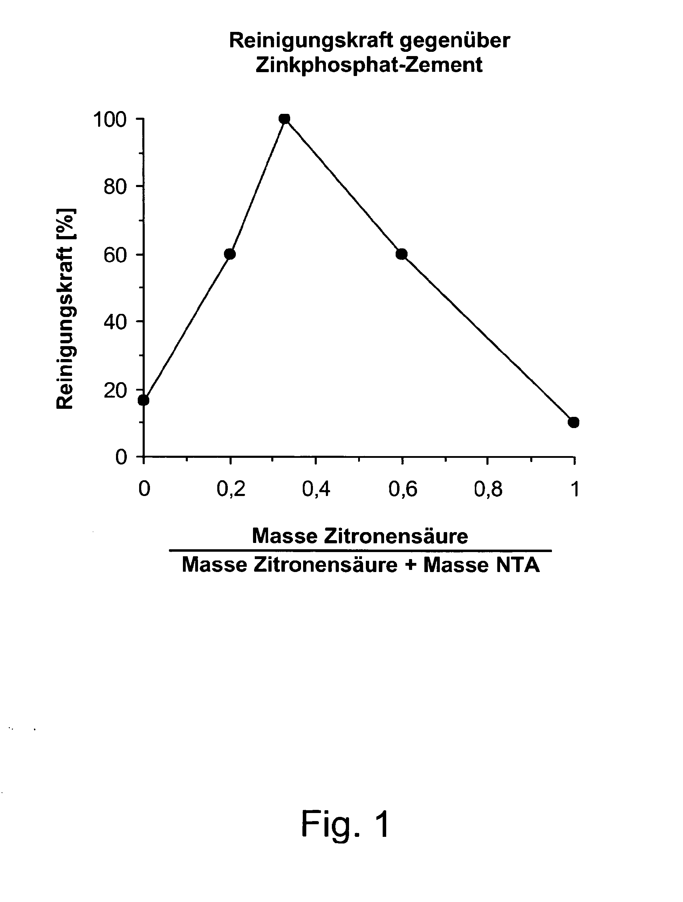

Bei einem Massenanteil an Zitronensäure von 0,33 (Zusammensetzung C) war die Reinigungskraft der Zusammensetzung ca. 6x so hoch wie beim ausschließlichen Einsatz von NTA (Zusammensetzung E) und ca. 10x so hoch wie beim ausschließlichen Einsatz von Zitronensäure (Zusammensetzung A). Diese synergistische Verstärkung der Reinigungskraft bei der Kombination der Chelatbildner NTA und Zitronensäure lässt sich auch anhand der Gleichung von Kull (Kull et al (1961) Appl Microbiol 9, 538) belegen. Fig. 1 illustriert die Ergebnisse.

### Beschreibung der Figur 1:

Auf der X-Achse ist der Massenanteil an Zitronensäure an der Nitrilotriacetat/Zitronensäure-Mischung aufgetragen. 1 bedeutet somit ausschließlich Zitronensäure (entsprechend Zusammensetzung A), 0 ausschließlich Nitrilotriacetat (entsprechend Zusammensetzung E). Auf der Y-Achse ist die auf den Maximalwert normierte Reinigungswirkung aufgetragen. Die synergistische Verstärkung der Reinigungswirkung ist bei einem Verhältnis Nitrilotriacetat : Zitronensäure von 2:1 am höchsten.

### Beispiel 21: Vergleich mit Reinigern aus dem Stand der Technik

Die Reinigungswirkung einer Lösung der tablettierten Zusammensetzung aus Beispiel 17, hergestellt durch Vermischen mit 4000 GT Wasser, wurde mit drei üblichen Reinigungsmitteln für Säure-Base-Zemente aus dem Stand der Technik verglichen. Gemäß Beispiel 18 wurden jeweils identische Prüfkörper aus Zinkphosphatzement (Poscal, VOCO) und Alginat (demedis) hergestellt und die Reinigungswirkungen quantifiziert.

Die Reinigungsmittel des Standes der Technik (I, II und III) hatten gemäß eigenen Untersuchungen folgende Zusammensetzungen (Vergleichsbeispiele):

**GT = Gewichtsteil**

| Produkt | NTA (GT) | EDTA (GT) | Tenside (GT) | Soda (GT) | Polyphosphat (GT) | Sonstige Inhalts-stoffe (GT)* |
|---|---|---|---|---|---|---|
| I | | | | 50 | 50 | 0 |
| II | | 10-15 | | | | ad 100 |
| III | 5-15 | | 6-20 | | | ad 100 |

### a) Den Herstellerangaben entsprechende Bedingungen

Entsprechend den Herstellerangaben wurden Lösungen hergestellt durch Vermischen von Produkt I mit 1250 GT Wasser und Produkt III mit 5000 GT Wasser. Produkt II lag bereits als Lösung vor und wurde unverdünnt eingesetzt. Diese Lösungen hatten die in der nachfolgenden Tabelle genannten pH-Werte (Standard-pH-Werte).

Ein Vergleich ihrer Reinigungswirkung unter diesen Bedingungen mit der Reinigungswirkung der gelösten erfindungsgemäßen Zusammensetzung brachte folgendes Ergebnis:

| Produkt | pH | Relative Reinigungs-wirkung Alginat | Relative Reinigungs-wirkung Zinkphosphat |
|---|---|---|---|
| I | 11 | 28,8 | 0,3 |
| II | 8,5 | 0,2 | 10,3 |
| III | 12 | 11,9 | 0,7 |
| gemäß Beispiel 17 | 8,5 | 100 | 100 |

Die Reinigungswirkungen sind dabei auf die Reinigungswirkung der gelösten erfindungsgemäßen Zusammensetzung normiert (Reinigungswirkung 100).

Die Reinigungswirkungen der Reinigungsmittel aus dem Stand der Technik waren unter den den Herstellerangaben entsprechenden Bedingungen (pH-Wert, Wirkstoffkonzentrationen in Lösung) niedriger als die der gelösten erfindungsgemäßen Zusammensetzung.

### b) Vereinheitlichte Bedingungen

Die als Feststoff vorliegenden Produkte des Standes der Technik (I und lll) wurden in demselben Verhältnis mit Wasser gemischt wie die erfindungsgemäße Zusammensetzung des Beispiels 17 (je 100 GT Produkt mit 4000 GT Wasser). Das als Lösung vorliegende Produkt II wurde unverdünnt eingesetzt und umfasste somit eine vergleichsweise hohe Konzentration an EDTA. Lösungen der Produkte I und III wurden durch Zugabe von HCl auf denselben pH-Wert wie die Lösung eingestellt, die sich beim Auflösen der erfindungsgemäßen Zusammensetzung ergibt (pH 8,5).

| Produkt | pH | Relative Reinigungs-wirkung Alginat | Relative Reinigungs-wirkung Zinkphosphat |
|---|---|---|---|
| I | 8,5 | 53,6 | 4,5 |
| II | 8,5 | 0,2 | 10,3 |
| III | 8,5 | 5,13 | 4,1 |
| gemäß Beispiel 17 | 8,5 | 100 | 100 |

Die Reinigungswirkungen sind dabei auf die Reinigungswirkung der gelösten erfindungsgemäßen Zusammensetzung normiert (Reinigungswirkung 100).

Die Reinigungswirkungen der Produkte I bis III (Stand der Technik) waren bei pH 8,5 schwächer als die der gelösten erfindungsgemäßen Zusammensetzung.

## Patentansprüche

1. Zusammensetzung zur Reinigung dentaler Instrumente von Säure-Base-Zementen, umfassend oder bestehend aus:
(a) Nitrilotriesessigsäure und/oder Nitrilotriacetat in einer Gesamtmenge von 20 bis 70 Gewichtsteilen, vorzugsweise 27,5 bis 70 Gewichtsteilen,
(b) Zitronensäure und/oder deren Salzen in einer Gesamtmenge von 15 bis 45 Gewichtsteilen,
(c) einem oder mehreren Alkalimetallcarbonaten und/oder -hydrogencarbonaten in einer Gesamtmenge von 0 bis 50 Gewichtsteilen,
(d) einem oder mehreren Tensiden in einer Gesamtmenge von 0 bis 5 Gewichtsteilen,
(e) einem oder mehreren Tablettierungshilfsstoffen in einer Gesamtmenge von 0 bis 10 Gewichtsteilen,
(f) einem oder mehreren Korrosionsinhibitoren in einer Gesamtmenge von 0 bis 5 Gewichtsteilen, und
(g) einem oder mehreren sonstigen Additiven in einer Gesamtmenge von 0 bis 55 Gewichtsteilen
wobei die Komponenten (a), (b), (c), (d), (e), (f) und (g) insgesamt in einer Menge von 100 Gewichtsteilen vorliegen, und
wobei die Komponenten so ausgewählt sind, dass der pH einer Lösung hergestellt durch Vermischen der Zusammensetzung mit 4000 Gewichtsteilen Wasser im Bereich von 7,0 bis 9,5 liegt.

2. Zusammensetzung nach Anspruch 1, wobei das Gewichtsverhältnis der Gesamtmenge von Nitrilotriessigsäure und Nitrilotriacetat zu der Gesamtmenge an Zitronensäure und deren Salzen in der Zusammensetzung größer ist als 1:1, vorzugsweise größer als 1,5 : 1.

3. Zusammensetzung umfassend oder bestehend aus:
(a) Nitrilotriessigsäure und/oder Nitrilotriacetat
(b) Zitronensäure und/oder deren Salze,
(c) einem oder mehreren Alkalimetallcarbonaten und/oder -hydrogencarbonaten in einer Gesamtmenge von 0 bis 50 Gewichtsteilen,
(d) einem oder mehreren Tensiden in einer Gesamtmenge von 0 bis 5 Gewichtsteilen,
(e) einem oder mehreren Tablettierungshilfsstoffen in einer Gesamtmenge von 0 bis 10 Gewichtsteilen,
(f) einem oder mehreren Korrosionsinhibitoren in einer Gesamtmenge von 0 bis 5 Gewichtsteilen und
(g) gegebenenfalls einem oder mehreren sonstigen Additiven,
wobei die Komponenten (a), (b), (c), (d), (e), (f) und (g) insgesamt in einer Menge von 100 Gewichtsteilen vorliegen, und
wobei das Gewichtsverhältnis der Gesamtmenge von Komponente (a) zu der Gesamtmenge an Komponente (b) in der Zusammensetzung größer ist als 1:1, vorzugsweise größer als 1,5 : 1,
wobei die Komponenten so ausgewählt sind, dass der pH einer Lösung hergestellt durch Vermischen der Zusammensetzung mit 4000 Gewichtsteilen Wasser im Bereich von 7,0 bis 9,5 liegt.

4. Zusammensetzung nach einem der vorangehenden Ansprüche, umfassend als Komponente (f) 0,1 bis 4 Gewichtsteile an einem oder mehreren Korrosionsinhibitoren.

5. Zusammensetzung nach einem der vorangehenden Ansprüche, umfassend als Komponente (c) 10 bis 25 Gewichtsteile an einem oder mehreren Alkalimetallcarbonaten und/oder -hydrogencarbonaten.

6. Zusammensetzung nach einem der vorangehenden Ansprüche, wobei die Zusammensetzung vorliegt als Tablette, Pulver, Granulat oder wässrige Lösung.

7. Zusammensetzung nach einem der vorangehenden Ansprüche, wobei die Zusammensetzung als Tablette vorliegt und als Komponente (e) 1 bis 10 Gewichtsteile an einem oder mehreren Tablettierungshiffsstoffen umfasst.

8. Als Tablette vorliegende Zusammensetzung nach einem der vorangehenden Ansprüche, umfassend oder bestehend aus:
(a) Nitrilotriessigsäure, Nitrilotriacetat oder deren Mischung in einer Gesamtmenge von 20 bis 70 Gewichtsteilen, vorzugsweise 30 bis 70 Gewichtsteilen,
(b) Zitronensäure, deren Salzen oder einer Mischung von Zitronensäure und deren Salzen in einer Gesamtmenge von 15 bis 45 Gewichtsteilen,
(c) einem oder mehreren Alkalimetallcarbonaten und/oder-hydrogencarbonaten in einer Gesamtmenge von 10 bis 50 Gewichtsteilen,
(d) einem oder mehreren Tensiden in einer Gesamtmenge von 0 bis 5 Gewichtsteilen,
(e) einem oder mehreren Tablettierungshilfsstoffen in einer Gesamtmenge von 1 bis 10 Gewichtsteilen,
(f) einem oder mehreren Korrosionsinhibitoren in einer Gesamtmenge von 0,1 bis 4 Gewichtsteilen, und
(g) gegebenenfalls einem oder mehreren sonstigen Additiven,
wobei die Komponenten (a), (b), (c), (d), (e), (f) und (g) insgesamt in einer Menge von 100 Gewichtsteilen vorliegen, und
wobei das Gewichtsverhältnis der Gesamtmenge von Nitrilotriacetat und Nitrilotriessigsäure zu der Gesamtmenge an Zitronensäure und ihren Salzen in der Zusammensetzung größer ist als 1:1, vorzugsweise größer als 1,5 : 1, und
wobei die Komponenten so ausgewählt sind, dass der pH einer Lösung hergestellt durch Vermischen der Zusammensetzung mit 4000 Gewichtsteilen Wasser im Bereich von 7,0 bis 9,5 liegt.

9. Als Tablette vorliegende Zusammensetzung, umfassend oder bestehend aus:
(A)
(a) Nitrilotriacetat-Natrium in einer Gesamtmenge von 24 bis 54 Gewichtsteilen, vorzugsweise 44 bis 54 Gewichtsteilen
(b) Zitronensäure, Salze der Zitronensäure oder einer Mischung von Zitronensäure und deren Salze in einer Gesamtmenge von 22,5 bis 26,5 Gewichtsteilen, vorzugsweise Zitronensäure alleine
(c) Natriumcarbonat in einer Gesamtmenge von 18 bis 22 Gewichtsteilen,
(d) C₁₆C₁₈-Fettalkoholethoxylate in einer Gesamtmenge von 0,45 bis 0,55 Gewichtsteilen,
(e) Polyethylenglykol 6000 in einer Gesamtmenge von 4,5 bis 5,5 Gewichtsteilen,
(f) 5-Methylbenzotriazol-Natrium in einer Gesamtmenge von 0,9 bis 1,1 Gewichtsteilen
oder
(B)
(a) Nitrilotriessigsäure in einer Gesamtmenge von 24 bis 54 Gewichtsteilen, vorzugsweise 34 bis 44 Gewichtsteilen
(b) Zitronensäure, Salze der Zitronensäure oder einer Mischung von Zitronensäure und deren Salze in einer Gesamtmenge von 19,2 bis 23,2 Gewichtsteilen, vorzugsweise Zitronensäure alleine
(c) Natriumcarbonat in einer Gesamtmenge von 32,5 bis 36,5 Gewichtsteilen,
(d) C₁₆C₁₈-Fettalkoholethoxylate in einer Gesamtmenge von 0,45 bis 0,55 Gewichtsteilen,
(e) Polyethylenglykol 6000 in einer Gesamtmenge von 4,5 bis 5,5 Gewichtsteilen,
(f) 5-Methylbenzotriazol-Natrium in einer Gesamtmenge von 0,9 bis 1,1 Gewichtsteilen.

10. Wässrige Reinigungsflüssigkeit für dentale Instrumente, umfassend
- eine gelöste oder dispergierte Zusammensetzung nach einem der Ansprüche 1 bis 9, wobei die Komponenten (a), (b), (c), (d), (e), (f) und (g) in einer Menge von insgesamt 100 Gewichtsteilen vorliegen, und
- Wasser in einer Menge von maximal 10000, bevorzugt 2000 bis 6000 Gewichtsteilen.

11. Verfahren zur Herstellung einer wässrigen Reinigungsflüssigkeit für dentale Instrumente, umfassend den folgenden Schritt:
- Herstellen einer Mischung umfassend eine Zusammensetzung nach einem der Ansprüche 1 bis 9, wobei die Komponenten (a), (b), (c), (d), (e), (f) und (g) in einer Menge von insgesamt 100 Gewichtsteilen vorliegen und Wasser in einer Menge von maximal 10000, bevorzugt 2000 bis 6000 Gewichtsteilen vorliegt.

12. Verwendung von Zitronensäure und/oder deren Salzen zur synergistischen Verstärkung der Säure-Base-Zementreste entfernenden Wirkung von Nitrilotriacetat, Nitrilotriessigsäure oder deren Mischung.

13. Verwendung von Nitrilotriacetat, Nitrilotriessigsäure oder deren Mischung zur synergistischen Verstärkung der Säure-Base-Zementreste entfernenden Wirkung von Zitronensäure und/oder deren Salzen.

14. Verwendung einer Zusammensetzung nach einem der Ansprüche 1 bis 9 oder einer wässrigen Reinigungsflüssigkeit nach Anspruch 10 als Mittel zur Entfernung von Verunreinigungen auf Basis von Säure-Base-Zementen.

15. Verfahren zur Entfernung von Verunreinigungen auf Basis von Säure-Base-Zementen, mit folgendem Schritt:
- Kontaktieren eines Gegenstandes, der eine Verunreinigung auf Basis von Säure-Base-Zementen trägt, mit einer wässrigen Reinigungsflüssigkeit nach Anspruch 10.

## Claims

1. Composition for cleaning dental instruments to remove acid-base cements, comprising or consisting of:
(a) nitrilotriacetic acid and/or nitrilotriacetate in a total amount of from 20 to 70 parts by weight, preferably 27.5 to 70 parts by weight,
(b) citric acid and/or salts thereof in a total amount of from 15 to 45 parts by weight,
(c) one or more alkali metal carbonates and/or bicarbonates in a total amount of from 0 to 50 parts by weight,
(d) one or more surfactants in a total amount of from 0 to 5 parts by weight,
(e) one or more tablet-making auxiliary substances in a total amount of from 0 to 10 parts by weight,
(f) one or more corrosion inhibitors in a total amount of from 0 to 5 parts by weight, and
(g) one or more other additives in a total amount of from 0 to 55 parts by weight wherein components (a), (b), (c), (d), (e), (f) and (g) in total are present in an amount of 100 parts by weight, and
wherein the components are chosen such that the pH of a solution prepared by mixing the composition with 4,000 parts by weight of water is in the range of from 7.0 to 9.5.

2. Composition according to claim 1, wherein the weight ratio of the total amount of nitrilotriacetic acid and nitrilotriacetate to the total amount of citric acid and salts thereof in the composition is greater than 1:1, preferably greater than 1.5:1.

3. Composition comprising or consisting of:
(a) nitrilotriacetic acid and/or nitrilotriacetate,
(b) citric acid and/or salts thereof,
(c) one or more alkali metal carbonates and/or bicarbonates in a total amount of from 0 to 50 parts by weight,
(d) one or more surfactants in a total amount of from 0 to 5 parts by weight,
(e) one or more tablet-making auxiliary substances in a total amount of from 0 to 10 parts by weight,
(f) one or more corrosion inhibitors in a total amount of from 0 to 5 parts by weight and
(g) optionally one or more other additives,
wherein components (a), (b), (c), (d), (e), (f) and (g) in total are present in an amount of 100 parts by weight, and
wherein the weight ratio of the total amount of component (a) to the total amount of component (b) in the composition is greater than 1:1, preferably greater than 1.5:1,
wherein the components are chosen such that the pH of a solution prepared by mixing the composition with 4,000 parts by weight of water is in the range of from 7.0 to 9.5.

4. Composition according to one of the preceding claims, comprising as component (f) 0.1 to 4 parts by weight of one or more corrosion inhibitors.

5. Composition according to one of the preceding claims, comprising as component (c) 10 to 25 parts by weight of one or more alkali metal carbonates and/or bicarbonates.

6. Composition according to one of the preceding claims, wherein the composition is present as a tablet, a powder, granules or an aqueous solution.

7. Composition according to one of the preceding claims, wherein the composition is present as a tablet and comprises as component (e) 1 to 10 parts by weight of one or more tablet-making auxiliary substances.

8. Composition according to one of the preceding claims present as a tablet, comprising or consisting of:
(a) nitrilotriacetic acid, nitrilotriacetate or a mixture thereof in a total amount of from 20 to 70 parts by weight, preferably 30 to 70 parts by weight,
(b) citric acid, salts thereof or a mixture of citric acid and salts thereof in a total amount of from 15 to 45 parts by weight,
(c) one or more alkali metal carbonates and/or bicarbonates in a total amount of from 10 to 50 parts by weight,
(d) one or more surfactants in a total amount of from 0 to 5 parts by weight,
(e) one or more tablet-making auxiliary substances in a total amount of from 1 to 10 parts by weight,
(f) one or more corrosion inhibitors in a total amount of from 0.1 to 4 parts by weight,
and
(g) optionally one or more other additives,
wherein components (a), (b), (c), (d), (e), (f) and (g) in total are present in an amount of 100 parts by weight, and
wherein the weight ratio of the total amount of nitrilotriacetate and nitrilotriacetic acid to the total amount of citric acid and its salts in the composition is greater than 1:1, preferably greater than 1.5:1, and
wherein the components are chosen such that the pH of a solution prepared by mixing the composition with 4,000 parts by weight of water is in the range of from 7.0 to 9.5.

9. Composition present as a tablet, comprising or consisting of:
(A)
(a) sodium nitrilotriacetate in a total amount of from 24 to 54 parts by weight, preferably 44 to 54 parts by weight,
(b) citric acid, salts of citric acid or a mixture of citric acid and salts thereof in a total amount of from 22.5 to 26.5 parts by weight, preferably citric acid by itself,
(c) sodium carbonate in a total amount of from 18 to 22 parts by weight,
(d) C₁₆C₁₈-fatty alcohol ethoxylates in a total amount of from 0.45 to 0.55 part by weight,
(e) polyethylene glycol 6000 in a total amount of from 4.5 to 5.5 parts by weight,
(f) 5-methylbenzotriazole-sodium in a total amount of from 0.9 to 1.1 parts by weight or
(B)
(a) nitrilotriacetic acid in a total amount of from 24 to 54 parts by weight, preferably 34 to 44 parts by weight,
(b) citric acid, salts of citric acid or a mixture of citric acid and salts thereof in a total amount of from 19.2 to 23.2 parts by weight, preferably citric acid by itself,
(c) sodium carbonate in a total amount of from 32.5 to 36.5 parts by weight,
(d) C₁₆C₁₈-fatty alcohol ethoxylates in a total amount of from 0.45 to 0.55 part by weight,
(e) polyethylene glycol 6000 in a total amount of from 4.5 to 5.5 parts by weight,
(f) 5-methylbenzotriazole-sodium in a total amount of from 0.9 to 1.1 parts by weight.

10. Aqueous cleaning liquid for dental instruments, comprising
- a dissolved or dispersed composition according to one of claims 1 to 9, wherein components (a), (b), (c), (d), (e), (f) and (g) are present in an amount of 100 parts by weight in total, and
- water in an amount of a maximum of 10,000, preferably 2,000 to 6,000 parts by weight.

11. Process for the preparation of an aqueous cleaning liquid for dental instruments, comprising the following step:
- preparation of a mixture comprising a composition according to one of claims 1 to 9, wherein components (a), (b), (c), (d), (e), (f) and (g) are present in an amount of 100 parts by weight in total and water is present in an amount of a maximum of 10,000, preferably 2,000 to 6,000 parts by weight.

12. Use of citric acid and/or salts thereof for synergistic intensification of the removing action of nitrilotriacetate, nitrilotriacetic acid or a mixture thereof on acid-base cement residues.

13. Use of nitrilotriacetate, nitrilotriacetic acid or a mixture thereof for synergistic intensification of the removing action of citric acid and/or salts thereof on acid-base cement residues.

14. Use of a composition according to one of claims 1 to 9 or of an aqueous cleaning liquid according to claim 10 as an agent for removal of contamination based on acid-base cements.

15. Method for the removal of contamination based on acid-base cements, with the following step:
- contacting of an object which carries contamination based on acid-base cements with an aqueous cleaning liquid according to claim 10.

## Revendications

1. Composition pour nettoyer des instruments dentaires des ciments acide-base, comprenant ou constituée de:
(a) acide nitrilotriacétique et/ou nitrilotriacétate en quantité totale de 20 à 70 parties en poids, de préférence 27,5 à 70 parties en poids,
(b) acide citrique et/ou ses sels en quantité totale de 15 à 45 parties en poids,
(c) un ou plusieurs carbonate/s de métal alcalin et/ou carbonate/s d'hydrogène alcalin en quantité totale de 0 à 50 parties en poids,
(d) un ou plusieurs tenside/s en quantité totale de 0 à 5 parties en poids,
(e) un ou plusieurs agent/s auxiliaire/s de formation de comprimés en quantité totale de 0 à 10 parties en poids,
(f) un ou plusieurs inhibiteur/s de corrosion en quantité totale de 0 à 5 parties en poids et
(g) un ou plusieurs autre/s additif/s en quantité totale de 0 à 55 parties en poids,
les composants (a), (b), (c), (d), (e), (f) et (g) représentant, tous ensemble, une quantité de 100 parties en poids et
les composants étant choisis de manière à ce que le pH d'une solution obtenue en mélangeant la composition à 4000 parties en poids d'eau soit compris entre 7,0 et 9,5.

2. Composition selon la revendication 1, le rapport en poids de la quantité totale d'acide nitrilotriacétique et de nitrilotriacétate à la quantité totale d'acide citrique et de ses sels dans la composition étant supérieur à 1:1, de préférence supérieur à 1,5:1.

3. Composition comprenant ou constituée de:
(a) acide nitrilotriacétique et/ou nitrilotriacétate,
(b) acide citrique et/ou ses sels,
(c) un ou plusieurs carbonate/s de métal alcalin et/ou carbonate/s d'hydrogène alcalin en quantité totale de 0 à 50 parties en poids,
(d) un ou plusieurs tenside/s en quantité totale de 0 à 5 parties en poids,
(e) un ou plusieurs agent/s auxiliaire/s de formation de comprimés en quantité totale de 0 à 10 parties en poids,
(f) un ou plusieurs inhibiteur/s de corrosion en quantité totale de 0 à 5 parties en poids et,
(g) le cas échéant, un ou plusieurs autre/s additif/s,
les composants (a), (b), (c), (d), (e), (f) et (g) représentant, tous ensemble, une quantité de 100 parties en poids et
le rapport en poids de la quantité totale de composant (a) à la quantité totale de composant (b) dans la composition étant supérieur à 1:1, de préférence supérieur à 1,5:1,
les composants étant choisis de manière à ce que le pH d'une solution obtenue en mélangeant la composition à 4000 parties en poids d'eau soit compris entre 7,0 et 9,5.

4. Composition selon l'une des revendications précédentes, comprenant, en tant que composant (f), 0,1 à 4 parties en poids d'un ou de plusieurs inhibiteur/s de corrosion.

5. Composition selon l'une des revendications précédentes, comprenant, en tant que composant (c), 10 à 25 parties en poids d'un ou de plusieurs carbonate/s de métal alcalin et/ou carbonate/s d'hydrogène alcalin.

6. Composition selon l'une des revendications précédentes, la composition se présentant sous forme de comprimé, poudre, granulé ou solution aqueuse.

7. Composition selon l'une des revendications précédentes, la composition se présentant sous forme de comprimé et comprenant, en tant que composant (e), 1 à 10 parties en poids d'un ou de plusieurs agents auxiliaire/s de formation de comprimés.

8. Composition selon l'une des revendications précédentes se présentant sous la forme de comprimé, comprenant ou constituée de:
(a) acide nitrilotriacétique, nitrilotriacétate ou leur mélange en quantité totale de 20 à 70 parties en poids, de préférence 30 à 70 parties en poids,
(b) acide citrique, ses sels ou un mélange d'acide citrique et de ses sels en quantité totale de 15 à 45 parties en poids,
(c) un ou plusieurs carbonate/s de métal alcalin et/ou carbonate/s d'hydrogène alcalin en quantité totale de 10 à 50 parties en poids,
(d) un ou plusieurs tenside/s en quantité totale de 0 à 5 parties en poids,
(e) un ou plusieurs agent/s auxiliaire/s de formation de comprimés en quantité totale de 1 à 10 parties en poids,
(f) un ou plusieurs inhibiteur/s de corrosion en quantité totale de 0,1 à 4 parties en poids et
(g) le cas échéant, un ou plusieurs autre/s additif/s,
les composants (a), (b), (c), (d), (e), (f) et (g) représentant, tous ensemble, une quantité de 100 parties en poids et
le rapport en poids de la quantité totale de nitrilotriacétate et d'acide nitrilotriacétique à la quantité totale d'acide citrique et de ses sels dans la composition étant supérieur à 1:1, de préférence supérieur à 1,5:1 1 et
les composants étant choisis de manière à ce que le pH d'une solution obtenue en mélangeant la composition à 4 000 parties en poids d'eau soit compris entre 7,0 et 9,5.

9. Composition se présentant sous la forme de comprimés, comprenant ou constituée· de:
(A)
(a) nitrilotriacétate de sodium en quantité totale de 24 à 54 parties en poids, de préférence 44 à 54 parties en poids,
(b) acide citrique, sels d'acide citrique ou un mélange d'acide citrique et de ses sels en quantité totale de 22,5 à 26,5 parties en poids, de préférence acide citrique seul,
(c) carbonate de sodium en quantité totale de 18 à 22 parties en poids,
(d) éthoxylates d'alcools gras C₁₆-C₁₈ en quantité totale de 0,45 à 0,55 parties en poids,
(e) polyéthylène glycol 6000 en quantité totale de 4,5 à 5,5 parties en poids,
(f) 5-méthylbenzotriazole de sodium en quantité totale de 0,9 à 1,1 parties en poids
ou
(B)
(a) acide nitrilotriacétique en quantité totale de 24 à 54 parties en poids, de préférence 34 à 44 parties en poids,
(b) acide citrique, sels d'acide citrique ou un mélange d'acide citrique et de ses sels en quantité totale de 19,2 à 23,2 parties en poids, de préférence acide citrique seul,
(c) carbonate de sodium en quantité totale de 32,5 à 36,5 parties en poids,
(d) éthoxylates d'alcools gras C₁₆-C₁₈ en quantité totale de 0,45 à 0,55 parties en poids,
(e) polyéthylène glycol 6000 en quantité totale de 4,5 à 5,5 parties en poids,
(f) 5-méthylbenzotriazole de sodium en quantité totale de 0,9 à 1,1 parties en poids.

10. Fluide de nettoyage aqueux pour instruments dentaires, comprenant
- une composition dissoute ou dispersée selon l'une quelconque des revendications 1 à 9, les composants (a), (b), (c), (d), (e), (f) et (g) représentant une quantité totale de 100 parties en poids et
- de l'eau en quantité maximale de 10000, de préférence 2000 à 6000, parties en poids.

11. Procédé de fabrication d'un fluide de nettoyage aqueux pour instruments dentaires, comprenant l'étape suivante:
- fabrication d'un mélange comprenant une composition selon l'une quelconque des revendications 1 à 9, les composants (a), (b), (c), (d), (e), (f) et (g) représentant une quantité totale de 100 parties en poids et l'eau représentant une quantité maximale de 10000, de préférence 2000 à 6000, parties en poids.

12. Utilisation d'acide citrique et/ou de ses sels pour le renforcement synergique de l'action éliminatrice de restes de ciments acide-base du nitrilotriacétate, de l'acide nitrilotriacétique ou de leur mélange.

13. Utilisation de nitrilotriacétate, d'acide nitrilotriacétique ou de leur mélange pour le renforcement synergique de l'action éliminatrice de restes de ciments acide-base de l'acide citrique et/ou de ses sels.

14. Utilisation d'une composition selon l'une quelconque des revendications 1 à 9 ou d'un fluide de nettoyage aqueux selon la revendication 10 comme moyen d'élimination de souillures à base de ciments acide-base.

15. Procédé d'élimination de souillures à base de ciments acide-base, comprenant l'étape suivante:
- mise en contact d'un objet portant une souillure à base de ciments acide-base avec un fluide de nettoyage aqueux selon la revendication 10.
